# EUROPEAN PATENT SPECIFICATION

(11) **EP 4 547 317 B1**
(45) Date of publication and mention of the grant of the patent: **09.12.2026**
(21) Application number: 23733982.5
(22) Date of filing: 21.06.2023
(51) Int. Cl.: A61N 1/36

(54) **SYSTEM FOR FEEDBACK CONTROL OF NEURAL STIMULATION**
SYSTEM ZUR RÜCKKOPPLUNGSSTEUERUNG DER NEURALEN STIMULATION
SYSTÈME DE COMMANDE DE RÉTROACTION DE STIMULATION NEURONALE

(30) Priority: 01.07.2022 US 202263357816 P; 09.08.2022 EP 22189490
(43) Date of publication of application: 07.05.2025
(73) Proprietor: BIOTRONIK SE & Co. KG, 12359 Berlin (DE)
(72) Inventor: BARU, Marcelo, Tualatin, Oregon 97062 (US); KIBLER, Andrew B., Lake Oswego, Oregon 97035 (US)
(74) Representative: Biotronik Corporate Services SE
(86) International application number: PCT/EP2023/066814
(87) International publication number: WO 2024/002822

(56) References cited:
- WO-A1-2020/124135
- US-A1- 2020 215 331
- US-A1- 2021 379 386
- US-A1- 2022 193 416

## Description

The present invention relates to a neurostimulation device for controlling a neurostimulation device.

Unlike Evoked Compound Action Potentials (ECAP) with tonic based stimulation (e.g. 40 Hz), recent studies (Gmel et al "The Effect of Spinal Cord Stimulation Frequency on the Neural Response and Perceived Sensation in Patients with Chronic Pain", Frontiers in Neuroscience, January 2021) show a decrease in ECAP amplitude and an increase in perceived stimulation strength with increasing stimulation frequency which indicates a heavy frequency coding component that outweighs the population coding at supra-threshold stimulation levels.

Feedback for closed-loop spinal cord stimulation (SCS) in prior art utilize ECAP-amplitude as control variable or some other variable derived from each individual ECAP or from the average of multiple ECAPs over several cycles. As disclosed in prior art, these measurements do not permit extracting the metrics needed to compute the perceived stimulation strength which is required to perform closed-loop control of SCS with higher stimulation frequencies. Advanced signal processing based on ECAPs are required.

In addition, existing closed-loop control implementations adjust amplitude in order to target a consistent ECAP measured amplitude, which must be empirically determined for each patient via testing in the clinic, varying strongly with the drugs the patient may be taking for pain control. These solutions do not provide for a method of determining the therapeutic window on a per-patient basis, for supra-perception nor sub-perception therapies.

Finally, known closed-loop SCS therapies are often limited to about 500 Hz as the measurement techniques utilized cannot prevent the stimulation pulse or its balancing component from interfering with the ECAP recording.

Particularly, US Patent No. 10,842,996 discloses a device for neurostimulation including an electrode structure for delivering stimulation pulses to a nerve as well as for processing and extracting evoked compound action potentials, wherein the electrode structure comprises at least a first anode, at least a second anode opposing the first anode and a plurality of cathodes arranged between said anodes, wherein said cathodes are asymmetrically arranged with respect to said at least first and second anode to permit evoked compound action potential sensing via the anode electrodes simultaneously with stimulation.

Furthermore, US 2020215331 A1 discloses a method of controlling a neural stimulus by use of feedback. The neural stimulus is applied to a neural pathway in order to give rise to an evoked action potential on the neural pathway. The stimulus is defined by at least one stimulus parameter. A neural compound action potential response evoked by the stimulus is measured. From the measured evoked response, a feedback variable is derived. A feedback loop is completed by using the feedback variable to control the at least one stimulus parameter value. The feedback loop adaptively compensates for changes in a gain of the feedback loop caused by electrode movement relative to the neural pathway.

US 2021/0379386 A1 discusses a neurostimulation device that has at least three stimulation electrodes configured to deliver an electrical stimulus to neural tissue. A control unit is configured to deliver a first stimulus phase in which a first stimulus electrode delivers a supra-threshold stimulus component, returned by at least two other of the stimulation electrodes. The control unit is further configured to deliver at least a second stimulus phase in which at least two of the stimulus electrodes deliver a sub-threshold stimulus component, returned by the first stimulus electrode.

Moreover, US 2022/0193416 A1 describes a method of applying a neural stimulus with an implanted electrode array, involving applying a sequence of stimuli configured to yield a therapeutic effect while suppressing psychophysical side effects. The stimuli sequence is configured such that a first stimulus recruits a portion of the fibre population, and a second stimulus is delivered within the refractory period following the first stimulus and the second stimulus being configured to recruit a further portion of the fibre population.

Based on the above, the problem to be solved by the present invention is to provide controlling for a neurostimulation device providing multiphase stimulation that allows a simple modelling of a transfer function and can be based on simple calibration data.

This problem is solved by a neurostimulation device according to claim 1.

According to claim, a neurostimulation device is disclosed, comprising a plurality of Z electrodes, Z is an integer number and equal or larger than 3. The neurostimulation device is configured to deliver in a cycle via each electrode of a group of N electrodes of said plurality of Z electrodes a set of electric pulses including one therapeutic electric pulse, and a number of N or N-1 charge balancing pulses. N is an integer number being smaller or equal to Z, wherein N is equal to 3 in case Z is equal to 3. The charge balancing electric pulses each have a polarity that is opposite a polarity of the therapeutic electric pulse. The integrated current delivered by the therapeutic electric pulse and charge balancing pulses is zero over time. The respective therapeutic electric pulse comprises an amplitude. The neurostimulation device is configured to record for the respective therapeutic electric pulse at least one ECAP signal, wherein the neurostimulation device comprises a closed-loop control system configured to update the amplitude of the therapeutic electric pulse based on said ECAP signal.

According to an embodiment of the present neurostimulation device, the current of each electric pulse is returned by the charge balancing pulses in the other N-1 electrodes.

Moreover, according to an embodiment, the ECAP signal is an antidromic ECAP signal and/or an orthodromic ECAP signal, wherein the amplitude of the therapeutic electric pulse is updated based on said ECAP signal by changing an absolute amplitude value or a percentage amplitude value.

Preferably, according to an embodiment of the present neurostimulation device, the closed-loop control system is configured to update the amplitude of the therapeutic electric pulse based on the ECAP signal in a way that one or more process variables D_{Total}; D_{Anti}; D_{Ortho} approaches a pre-defined set value D_{PR}, wherein the control system is configured to calculate an actual value of the process variable using the antidromic and/or orthodromic ECAP signals.

According to an embodiment of the present neurostimulation device, the neurostimulation device comprises a plurality of Z electrodes, wherein Z is an integer number and equal or larger than 3, the neurostimulation device being configured to deliver in a cycle via each electrode of a group of N electrodes of said plurality of Z electrodes, wherein N is an integer number being smaller or equal to Z, wherein N is equal to 3 in case Z is equal to 3, a set of electric pulses as follows:
- Each electrode undergoes a therapeutic electric pulse of amplitude I₁ I₂ ... I_{N} in phases at a frequency f and charge balancing pulses of opposite polarity;
- The current of each therapeutic electric pulse is in part or in whole returned by the charge balancing pulses in the other N-1 electrodes;
- The integrated average current delivered by each therapeutic electric pulse and charge balancing pulses, along with an optional final active or passive balance phase is zero over time;
   and wherein
- the neurostimulation device is configured to record for each respective electric pulse an antidromic evoked compound action potential (ECAP) signal and/or an orthodromic ECAP signal, and wherein
- the neurostimulation device comprises a closed-loop control system configured to adjust a parameter of the respective therapeutic electric pulse(s) so that a process variable associated with that therapeutic electric pulse phase approaches a predefined set value, wherein the control system is configured to determine an actual value of the process variable using the antidromic and/or orthodromic ECAP signals.

According to a preferred embodiment of the neurostimulation device, the control system is configured to subtract the actual value of the process variable from a pre-defined set value D_{PR} to calculate an error.

Furthermore, according to a preferred embodiment of the neurostimulation device, the control system comprises a controller configured to add an increment to the amplitude of the respective therapeutic electric pulse for updating the amplitude of the respective therapeutic electric pulse, wherein the adjustment is proportional to the error multiplied with a factor 1/m.

Further, according to a preferred embodiment of the neurostimulation device, the factor 1/m is the inverse of a slope m of an approximation of a process variable -amplitude transfer function.

According to a further preferred embodiment of the neurostimulation system, the control system is configured to approximate a process variable - therapeutic electric pulse amplitude transfer function that assigns a value of the process variable D_{Process} (i.e. corresponding therapy dose) to each value of the therapeutic electric pulse amplitude i_{TPE}, by at least a first linear portion and a subsequent second linear portion, the first linear portion comprising a first slope m₁ and the second linear portion comprising a different second slope m₂, wherein the first portion includes values of the process variable D_{Process} smaller or equal to a threshold, and the second portion includes values of the process variable D_{Process} above the threshold.

Furthermore, according to a preferred embodiment of the neurostimulation device, the control system is configured to empirically estimate the second slope m₂. Particularly, according to an embodiment, the process variable D_{Process} can be the antidromic portion D_{Anti} or orthodromic portion D_{Ortho}, or total therapy dose D_{Total}. Further, k is a constant that can be determined empirically, and i_{TPE} is the actual amplitude of the respective therapeutic electric pulse.

Further, according to a preferred embodiment of the neurostimulation device, the control system comprises a processing unit configured to select as said slope m the first slope m₁ in case the actual value of the process variable is below or equal to the D_{PR} threshold, and to select as said slope m the second slope m₂ in case the actual value of the process variable is above the D_{PR} threshold.

Furthermore, in a preferred embodiment of the neurostimulation device, the neurostimulation device (e.g. the closed-loop control system) is configured to remove a remnant stimulation artefact from the respective (antidromic or orthodromic) ECAP signal prior to calculating the actual value of the process variable D_{Process}, wherein preferably the closed-loop control system is configured to subtract a remnant stimulation artefact template from the respective ECAP signal for removing the remnant stimulation artefact.

Further, according to a preferred embodiment of the neurostimulation device, the neurostimulation device comprises at least two electronic circuit front-ends for recording the antidromic and/or orthodromic ECAP signals.

Furthermore, according to a preferred embodiment of the neurostimulation device, the neurostimulation device (e.g. the closed-loop control system) is configured to convert in a calibration cycle a differential output of each recording front-end to a single-ended output, digitize the single-ended output, and store the digitized single-ended output, wherein the neurostimulation device is configured to generate and fit a remnant stimulation artifact (SA) template to the respective digitized single-ended calibration output, and wherein during recording of the respective (antidromic and/or orthodromic) ECAP signal, the neurostimulation device is configured to output the respective remnant stimulation artifact template via a digital-to-analog converter to yield an analog template and to subtract the analog template from the single-ended output (containing the respective remnant stimulation artifact and ECAP signal) for generating the respective ECAP signal with removed remnant stimulation artifact.

Further, in a preferred embodiment of the neurostimulation device, the neurostimulation device (e.g. the closed-loop control system) is configured to convert a differential output of each recording front-end to a single-ended output, to digitize an initial remnant stimulation artifact comprised therein (in the single-ended output) by means of an analog-to-digital converter and to store it as an initial template in a memory, wherein the neurostimulation device (e.g. the closed-loop control system) configured to iteratively update the initial template by subtracting the single-ended output or a fraction of the single ended output (containing the remnant stimulation artefact and ECAP signal) from an analog conversion of the stored template generated by an digital-to-analog converter to yield a present template until an incoming remnant stimulation artifact and the present template converge within the resolution of the analog-to-digital converter and digital-to-analog converter, wherein the calculated template corresponding to each stimulation phase is then subtracted (as a final template) synchronized with each respective therapeutic electric pulse from all following recorded ECAP signals for that stimulation phase.

Particularly, utilizing the value of the mean of the rectified and time-averaged ECAP signal, squared, has the benefit that the latter is proportional to the firing frequency or number of active nerve fibers and thus decodes the frequency-coded perceived stimulation strength, i.e. the process variable D_{Process} for closed-loop control as per this invention disclosure.

Furthermore, according to a preferred embodiment of the neurostimulation system according to the present invention, the process variable D_{Process} corresponds to a total therapy dose D_{Total}, wherein the control system is configured to calculate an actual value of the process variable from the antidromic and orthodromic ECAP signals after removal of the remnant stimulation artifacts of the antidromic and orthodromic ECAP signals, by fully-wave rectifying the respective (antidromic or orthodromic) ECAP signal, averaging the respective ECAP signal (e.g. by bin integration), generating a weighted sum of the averaged antidromic ECAP signals, wherein the weight (k_{Anti}) account for different spacings between electrodes used for recording the respective antidromic ECAP signal; generating a weighted sum of the averaged orthodromic ECAP signals, wherein the weight (k_{Ortho}) account for different spacings between electrodes used for recording the respective orthodromic ECAP signal, adding the two weighted sums to generate a final sum, and squaring the final sum which generates the actual value of the process variable of the total therapy dose D_{Total}.

Furthermore, according to a preferred embodiment of the neurostimulation system, the process variable D_{Process} corresponds to an antidromic therapy sensation dose (D_{Anti}), wherein the control system is configured to calculate an actual value of the process variable (i.e. of the antidromic therapy sensation dose D_{Anti}) from the antidromic ECAP signals after removal of the remnant stimulation artifacts from the antidromic ECAP signals, by fully-wave rectifying the respective antidromic ECAP signal, averaging the respective antidromic ECAP signal (e.g. by bin integration), generating a weighted sum of the averaged antidromic ECAP signals, wherein the weights (k_{Anti}) account for different spacings between electrodes used for recording the respective antidromic ECAP signal, and squaring the weighted sum which generates the actual value of the process variable D_{Process} (here of the antidromic therapy sensation dose D_{Anti}).

Furthermore, according to a preferred embodiment of the neurostimulation system, the process variable D_{Process} corresponds to an orthodromic therapy sensation dose (D_{Ortho}), wherein the control system is configured to calculate an actual value of the process variable (i.e. of the orthodromic therapy sensation dose D_{Ortho}) from the orthodromic ECAP signals after removal of the remnant simulation artifacts from the orthodromic ECAP signals, by fully-wave rectifying the respective orthodromic ECAP signal, averaging the respective orthodromic ECAP signal (e.g. by bin integration), generating a weighted sum of the averaged orthodromic ECAP signals, wherein the weights (k_{Ortho}) account for different spacings between electrodes used for recording the respective orthodromic ECAP signal, and squaring the weighted sum which generates the actual value of the process variable D_{Process} (here of the orthodromic therapy sensation dose D_{Ortho}).

According to an embodiment of the present inventive neurostimulation device, the neurostimulation device is further configured to determine and deliver individual amplitudes per phase, and/or to define individual remnant stimulation artefact templates per phase in dependency of the recorded ECAP signal, and/or to apply individual control loops for closed-loop control per phase having individual process variables D_{To}tal; D_{Anti}; D_{Ortho} approaches a pre-defined set value D_{PR}.

Moreover, a not claimed method for controlling neurostimulation is disclosed, wherein the method uses a plurality Z of electrodes, wherein Z is an integer number and equal or larger than 3. In a cycle, via each electrode of a group of N electrodes of the plurality of Z electrodes, a set of electric pulses is generated including at least one therapeutic electric pulse and a number of N-1 charge balancing pulses. N is an integer number being smaller or equal to Z, wherein N is equal to 3 in case Z is equal to 3. The charge balancing electric pulses each have a polarity that is opposite a polarity of the therapeutic electric pulse. The integrated current delivered of the therapeutic electric pulse and charge balancing pulses is zero over time. The respective therapeutic electric pulse comprises an amplitude. The method comprises recording for the respective therapeutic electric pulse at least one ECAP signal and updating the amplitude of the therapeutic electric pulse based on said ECAP signal.

According to yet another not claimed example, a method for controlling neurostimulation using a plurality Z of electrodes is disclosed, wherein Z is an integer number and equal or larger than 3, wherein in a cycle, via each electrode of a group of N electrodes of said plurality of Z electrodes, wherein N is an integer number being smaller or equal to Z, wherein N is equal to 3 in case Z is equal to 3, a set of electric pulses is generated including one therapeutic electric pulse, and a number of N-1 charge balancing pulses, wherein the charge balancing electric pulses each have a polarity that is opposite a polarity of the therapeutic electric pulse, and wherein for the respective therapeutic electric pulse an antidromic ECAP signal and/or an orthodromic ECAP signal (evoked compound action potential) is recorded, and wherein a closed-loop control system adjusts the amplitude of the respective therapeutic electric pulse so that a process variable approaches a pre-defined set value, wherein an actual value of the process variable is calculated using the antidromic and/or orthodromic ECAP signals.

It is understood that, where applicable, features described in association with the inventive neurostimulation device are transferrable to the inventive method described herein and vice versa.

In the following, detailed embodiments and features of the present invention will be described below with reference to the Figures, wherein
- Fig. 1: shows an embodiment of a neurostimulation system according to the present invention;
- Fig. 2: shows an embodiment of recording of ECAPs during a multiphase (a.k.a. Rotating Electrodes) stimulation using two electronic circuit front-ends;
- Fig. 3: shows an exemplary illustration of removing a remnant SA based on template subtraction according to an embodiment of the present invention;
- Fig. 4: shows schematic block diagrams for removing remnant stimulations artifacts from recorded ECAPs according to two different embodiments of a neurostimulation system according to the present invention;
- Fig. 5: shows a block diagram for calculating a total therapy dose D_{Total} as well as antidromic and orthodromic therapy sensation doses D_{Anti}, D_{Ortho} respectively according to an embodiment of the present invention;
- Fig. 6: illustrates the therapy dose range applied during multiphase stimulation according to a preferred embodiment of the present invention;
- Fig. 7: shows a process variable - therapeutic electric pulse amplitude transfer function that can be employed by a closed-loop control system of a neurostimulation system according to a preferred embodiment of the present invention; and
- Fig. 8: shows a schematic block diagram of a closed-loop control system of a neurostimulation system according to a preferred embodiment of the present invention.

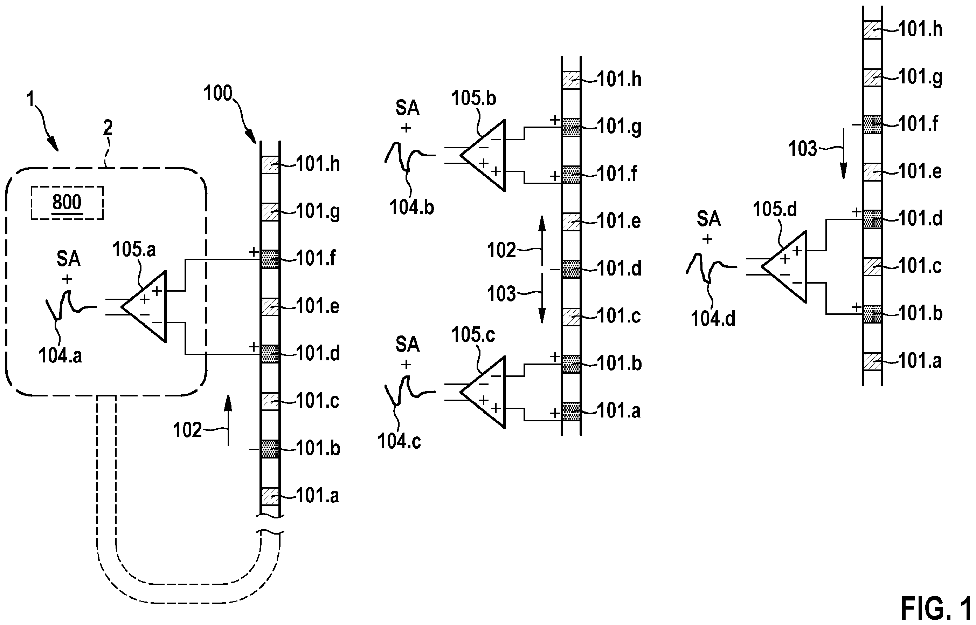

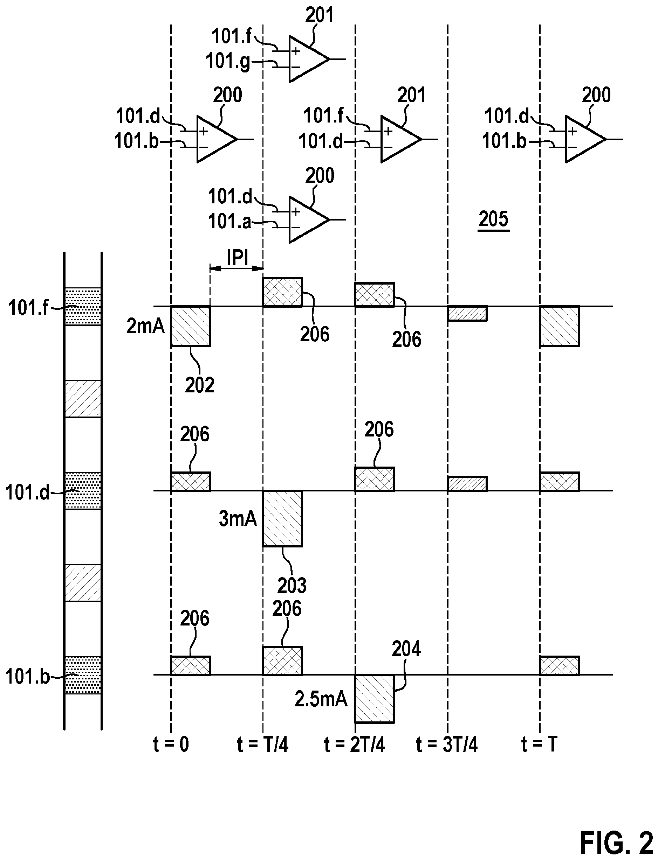

According to a preferred embodiment of the neurostimulation system 1 according to the present invention, the neurostimulation system 1 can comprise a preferably implantable pulse generator (IPG) 2 connected to one or more percutaneous or paddle leads 100 with a plurality Z of electrodes 101 (i.e. 101.a to 101.h, Z = 8 in this case) as shown in Fig. 1. As an example, the neurostimulation system 1 (particularly said IPG 2) is configured to deliver in a cycle T (cf. also Fig. 2) via each electrode 101.b, 101.d, 101.f of a group of N electrodes (N = 3 in this case) a set of electric pulses including one therapeutic electric pulse 202, 203, 204, and a number of (N-1) charge balancing pulses 206, wherein the charge balancing electric pulses 206 each have a polarity that is opposite a polarity of the therapeutic electric pulse 202, 203, 204, and wherein the respective therapeutic electric pulse comprises different amplitude(s) i_{TPE}. Preferably, the therapeutic electric pulses 202, 203, 204 (cf. Fig. 2) are generated in a subsequent fashion, wherein each therapeutic electric pulse 202, 203, 204 are accompanied by simultaneous charge balancing pulses 206 of the other (N-1) electrodes. Such a delivery of electric pulses is denoted as multiphase therapy and is disclosed, for example, in US Patent No. 10,870,000.

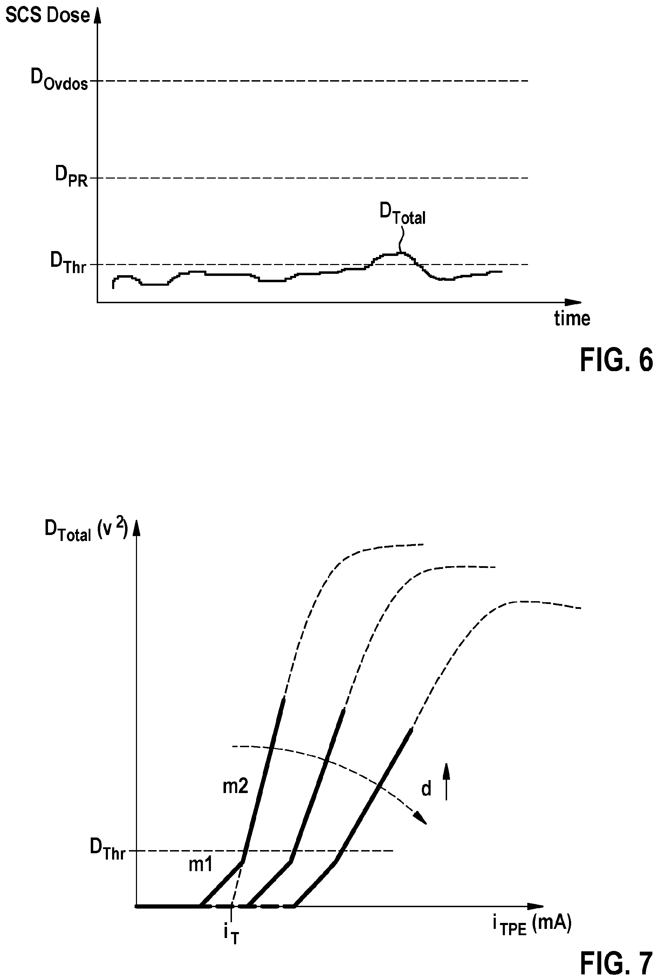

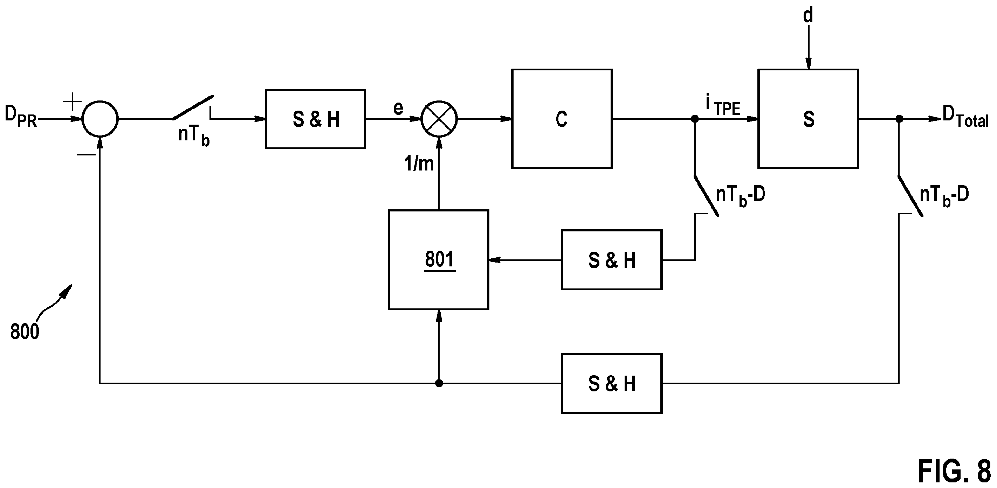

Particularly, the neurostimulation system 1 is configured to record, for the respective therapeutic electric pulse, an antidromic evoked compound action potential (ECAP) signal 104.a, 104.b and/or an orthodromic ECAP signal 104.c, 104.d, which will be described in more detail below. Further, the neurostimulation system 1 comprises a closed-loop control system 800 (cf. also Fig. 8) configured to adjust the amplitude i_{TPE} of the respective therapeutic electric pulse 202, 203, 204 so that an actual value of a process variable D_{Process}, preferably the total therapy dose D_{Total}, approaches a pre-defined set value D_{PR} (cf. also Fig. 6) wherein the control system 800 is configured to calculate an actual value of the process variable D_{Process} using the antidromic and/or orthodromic ECAP signals 104.a, 104.b, 104.c, 104.d.

Multiphase stimulation operates in an antidromic 102 or local field potential fashion but its orthodromic 103 effects are unknown. In a preferred embodiment, the system according to the present invention records ECAPs 104 synchronized with each therapeutic electric pulse of the multiphase therapy as shown in Fig. 1 for the case of a percutaneous lead 100 with eight electrodes 101.a, ..., 101.h (Z = 8 ) and therapeutic electric pulses delivered via electrodes 101.b, 101.d and 101.f (N = 3, a similar description can be done with the electrodes 101 of a paddle lead column). For example, when electrode 101.b provides the therapeutic electric pulse, recording front-end 105.a records an antidromic ECAP 104.a plus remnant SA via electrodes 101.d, 101.f (electrodes 101.c, 101.e are skipped). When electrode 101.d delivers the therapeutic electric pulse instead, recording of antidromic ECAP 104.b plus remnant SA occurs via front-end 105.b and electrodes 101.f, 101.g whereas orthodromic ECAP 104.c plus remnant SA is recorded via front-end 105.c and electrodes 101.a, 101.b. Finally, to complete the multiphase cycle, when electrode 101.f delivers the therapeutic electric pulse, orthodromic ECAP 104.d plus remnant SA is recorded via front-end 105.d and electrodes 101.b, 101.d.

Recording front-ends 105 are preferably fully-differential to reject the voltage swing in the recording electrodes 101 that undergo similar excursions during therapeutic electric pulse delivery and other external noise sources that may be present during recording. Different implementations are possible for recording front-ends 105. For example, front-ends 105.a and 105.b may be the implemented by the same circuitry whereas the same can occur for front-ends 105.c and 105.d. When not recording, each front-end 105 is preferably blanked at the input.

Considering an embodiment of the present invention comprising two recording front-ends 200, 201; the connection/disconnection of these recording front-ends during the different therapeutic electric pulses is illustrated in Fig. 2. As an example, when a therapeutic electric pulse 202 of 2 mA is delivered in electrode 101.f, recording front-end 200 will have its non-inverting input connected to electrode 101.d and the inverting input connected to electrode 101.b. The recording continues during each inter-pulse interval (IPI) following a therapeutic electric pulse. When a therapeutic electric pulse 203 of 3 mA is to be delivered in electrode 101.d, recording front-end 200 will switch its inverting input connection to electrode 101.a. Recording front-end 201 will also be connected at this time with its non-inverting input connected to electrode 101.f and the inverting input connected to electrode 101.g. Finally, when it is time to deliver therapeutic electric pulse 204 in electrode 101.b of 2.5 mA, the recording front-end 200 output is blanked and the inverting input of recording front-end 201 switched and connected to electrode 101.d. During the auxiliary charge balance phase 205 recording does not occur.

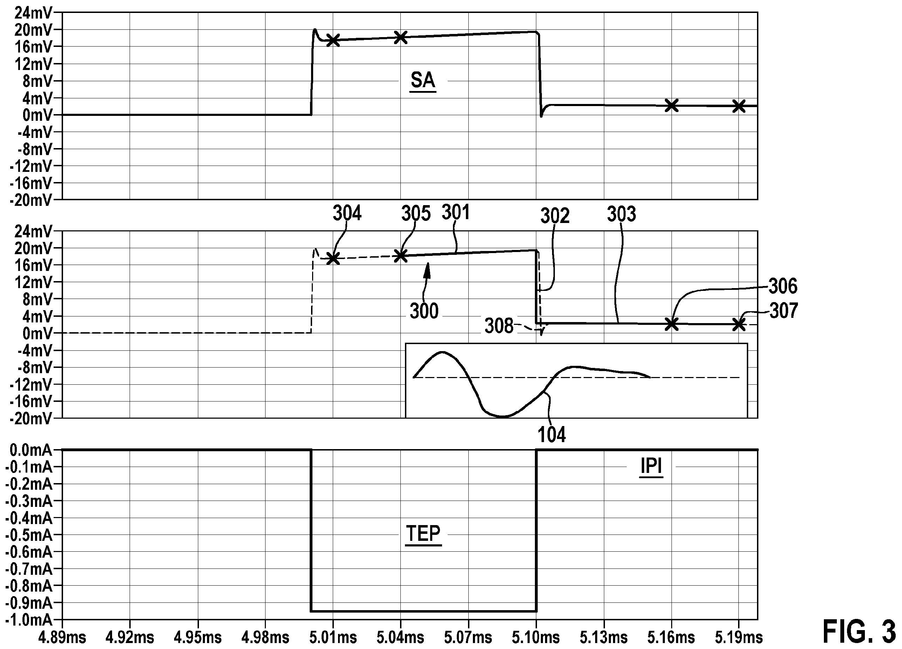

To compute the signal(s) for feedback control of closed-loop SCS, the remnant SA needs to be removed first from each ECAP 104 signal. Given the inter-electrode 101 spacing, each signal 104 will appear some tens of µs after the therapeutic electric pulse phase has settled to its plateau amplitude. In a preferred embodiment, as indicated in Fig. 3, the remnant SA can be fitted to an artifact template 300 composed of slope 301 during the therapeutic electric pulse when 104 is occurring, and ohmic drop 302 when therapeutic electric pulse finishes, followed by another slope 303 during the IPI. Slope 301 can be determined from samples 304, 305 which preferably occur before the ECAP signal 104 appears. The slope 303, on the other hand, can be determined from samples 306, 307 which are located at the end of the IPI before the next therapeutic electric pulse. Selecting samples this way minimizes the influence of the actual ECAP 104 (not to scale in Fig. 3) on the remnant SA to be removed. The ohmic drop 302 can extrapolated from the intersection in time with the end of the therapeutic electric pulse.

Other non-linear SA templates, e.g. exponential decay combined with a linear slope, are also possible to be fitted as preferred embodiments.

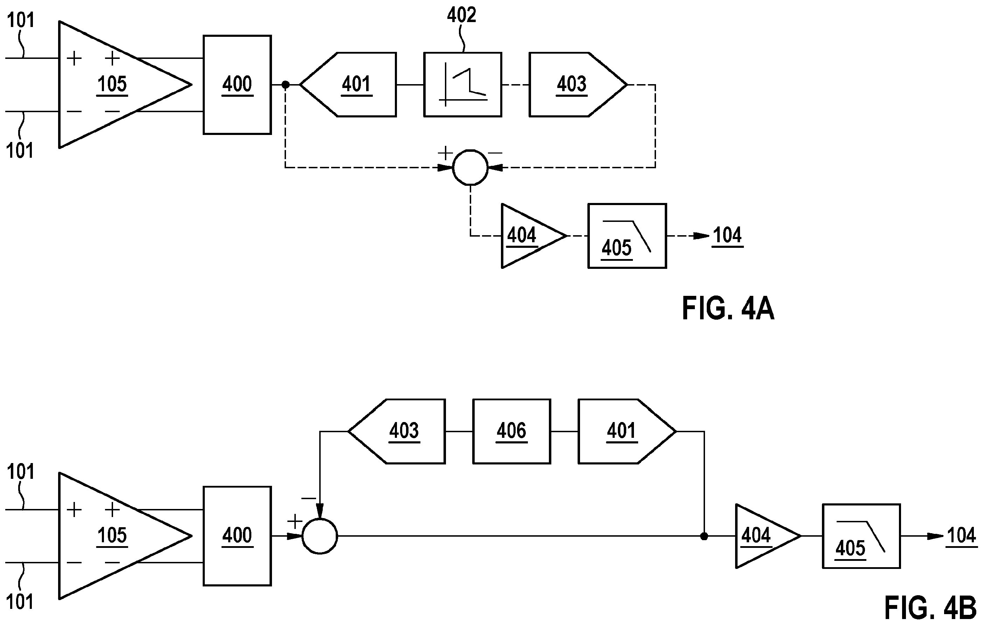

A preferred final processing embodiment to remove the remnant SA is illustrated in Fig. 4.a and it is based on template subtraction. In a full "calibration" cycle of therapeutic electric pulses (solid lines path in Fig. 4.a), the differential output of each recording front-end 105 is converted to single-ended by block 400 (fully-differential chain processing is also possible), digitized via analog-to-digital converter (ADC) 401 (anti-aliasing filter in between not shown for simplicity), and stored in IPG memory 406. The ADC 401 output is used by digital signal processing 402 to generate a fitted remnant SA 300 template which will be output via digital-to-analog converter (DAC) 403 (synchronized with each therapeutic electric pulse) for cancellation during actual ECAP recording.

During ECAP recording (dotted path line in Fig. 4.a), the analog output of block 400 (containing amplified SA and ECAP signal 104) is subtracted with DAC 403 output and such difference further amplified by block 404 and low-pass filtered via block 405 to generate the ECAP 104 signal. In a preferred embodiment, the low-pass filter 405 corner frequency is 5 kHz. This eliminates the SA spikes 308 that were not removed by the fitted remnant SA 300 template signal subtraction while also minimizing circuit noise. High-pass filtering, with a corner frequency of 300 Hz, is also implemented (not shown) preferably at the recording front-end 105. This allows AC coupling to electrodes 101 for ECAP recording purposes, minimizing circuit noise and contribution from external noise sources. In a preferred embodiment, the band-pass filtering of 300 Hz to 5 kHz is second order.

In an alternative embodiment for remnant SA subtraction, an iterative calibration hardware loop with ADC 401 and DAC 403 could be employed instead as shown Fig. 4.b. In this embodiment, with DAC 403 output as zero, an initial remnant SA is digitized by ADC 401 and stored as an initial template in IPG memory 406. Then the template is iteratively updated subtracting the output of analog block 400 (containing amplified SA and ECAP signal 104) from the present template until the incoming remnant SA and stored template converge within the resolution of the ADC 401 and DAC 403. The final template is then subtracted (synchronized with each therapeutic electric pulse) from all following ECAP recording and the result amplified by block 404 and low-pass filtered by block 405. Subtracting the ECAP signal 104 during remnant SA template saving is avoided by selecting the correct gain for front-end 105, and the correct ADC 401 and DAC 403 resolutions. Fig. 4.b is preferably utilized if the remnant SA is order of magnitudes larger than the ECAP signal 104. If the stimulation therapy is changed, or body postures changes occur, the calibration cycle needs to be repeated to save the new remnant SA template. Hence, preferably, re-calibration occurs periodically and automatically. These applies to both embodiments of Fig. 4.

As it can be appreciated by people skilled in the art, other embodiments for remnant SA subtraction at the front-end 105 can be employed. For example, an on-chip digital filter with adjustable coefficients (mimicking the electrode-tissue interface response) can be adjusted in a calibration phase to replicate the remnant SA via DAC 403 and later subtracted at the input of the recording front-end 105 during ECAP recording. US Patent 10,842,996 also discloses SA subtraction for ECAP recording.

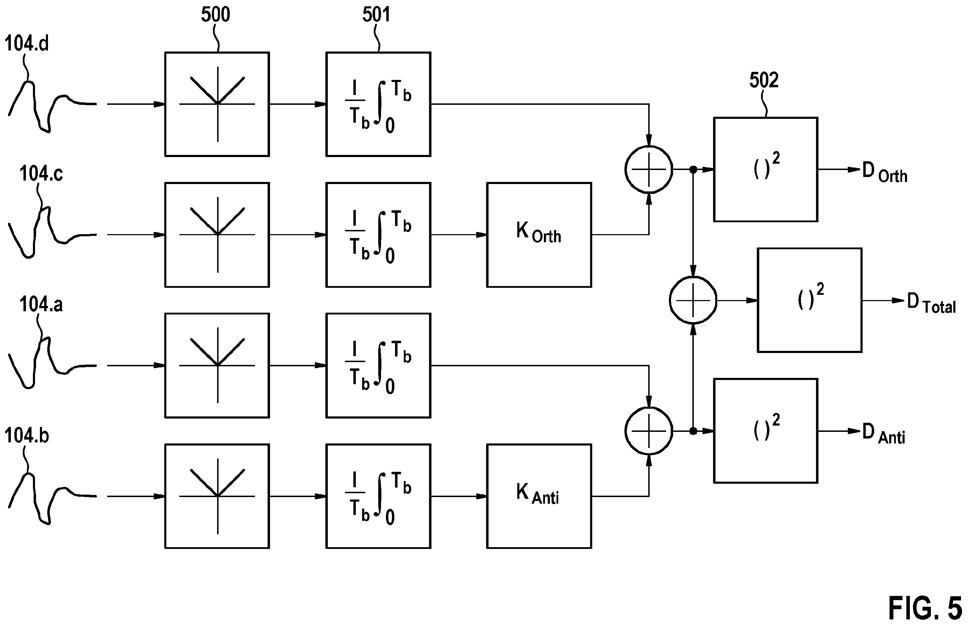

Once the remnant SAs have been removed, an orthodromic therapy sensation dose D_{Ortho}, an antidromic therapy sensation dose D_{Anti} and a total therapy dose D_{Total} can be computed as shown Fig. 5. First each signal 104 is fully-wave rectified 500, followed by a bin-integration 501. The bin duration T_{b} may be dependent on the multiphase repeating period T (see Fig. 2). For example, T_{b} may be equal to 10 x T (i.e., ten cycles average). Since the signals 104.b and 104.c are recorded with adjacent electrodes 101, they may record a slight smaller ECAP amplitude compared to 104.a and 104.d respectively (that are measured with a skipped electrode 101) so multiplying factors k_{Ortho} and k_{Anti} account for that difference before the corresponding antidromic and orthodromic processed 104 signals can be added. Finally, the orthodromic therapy sensation dose D_{Ortho}, the antidromic therapy sensation dose D_{Anti} and the total therapy dose D_{Total} can be computed by squaring 502 the proper addition of processed 104 signals.

In a preferred embodiment, feedback control for closed-loop multiphase SCS uses the total therapy dose D_{Total} as variable. Alternatively, it may use either the orthodromic therapy sensation dose D_{Ortho} or the antidromic therapy sensation dose D_{Anti}. multiphase SCS therapy may have three SCS Dose zones as illustrated in Fig. 6. Zone 0 to D_{Thr} defines the sub-perception zone where multiphase is preferred to be run, preferably somewhere between 60% to 80% of D_{Thr}. But D_{Total} may be permitted to go into the "perception" zone defined between the threshold D_{Thr} and an over-dose threshold D_{Ovdos}. Dose level D_{PR} may be set by the patient/clinician via a patient remote/clinician programmer.

Further, according to a preferred embodiment of the present invention, a relationship between the total therapy dose D_{Total} and for example the TPE amplitude i_{TPE} that can be used for sensation control is shown in Fig. 7. The curve is a sigmoid but for the purpose of SCS closed-loop control it can be linearized in two regions of interest. The first region is represented by slope m1 which can be assumed to remain constant as the distance d between the electrode-fibers vary with patient postures. In this region temporal neural integration and facilitatory effects of multiphase stimulation define the recruitment. The threshold D_{Thr} (see Fig. 6) can be empirically determined in each patient for the most sensitive body position (e.g. supine position). Slope m2, on the other hand, is not constant, but it can be assumed the product of *m*₂ · *i_{T}* where i_{T} is the intercept of the linear slopes m₂ with the x-axis (i.e. D_{Total} equals zero), is equal to a constant k that can be empirically determined. Hence, when the total therapy dose D_{Total} exceeds D_{Thr}, slope m₂ can be estimated as (*D_{TotaL} + k*)/*i_{TPE}* for the present condition.

In a preferred embodiment (cf. Fig. 8), the feedback loop 800 comprises a stimulator S, which delivers multiphase therapy, as well as circuitry for the non-linear signal processing to compute the process variable D_{Process} (preferably total therapy dose D_{Total}). A preferred input change for stimulator S is the TPE amplitude i_{TPE} of the multiphase therapy (or a percentage when different amplitudes are utilized, see Fig. 2). As mentioned above, the stimulator's S process variable - therapeutic electric pulse amplitude transfer function is variable since the electrode-fibers distance varies with posture changes, breathing, heart beats, etc. (d input in Fig. 7 and Fig. 8).

Particularly, block 801 determines which coefficient m₁ or m₂ to use to multiply error e (i.e. difference between actual process variable D_{Process} and target dose level D_{PR}) by the inverse of the corresponding slope, i.e. 1/m with m either m₁ or estimated m₂ as (*D_{TotaL} + k*)/*i_{TPE}* as described before.

Control block C preferably ramps up i_{TPE} from a minimum value to minimize perception. Each period (*n · T_{b} - D),* where *n* ≥ 1 and *D* a small delay (may be IPI), D_{Process} is calculated and both i_{TPE} and D_{Process} sampled and held (S&H). From this info block 801 computes 1/m (starting point 1/m1) and at each period *n* · *T_{b}* the error e is sampled and held and multiplied by 1/m so block C can calculate the next i_{TPE} to apply being *i_{TPE}*[(*n* + 1) . *T_{b}*] = *i_{TPE}*[*n* ·*T_{b}*] *+ e*[*n · T_{b}*]/*m,* wherein the square brackets denote the respective argument of the functions i_{TPE} and e.

To attenuate heartbeat noise, in another preferred embodiment, closed-loop multiphase SCS may be delivered outside the heart QT interval. Heart rate can be sensed between an unused electrode 101 and the IPG case as taught e.g. in US Patents 10,183,168 and 10,842,996.

Although the embodiments described before were based on concurrent multiphase SCS and ECAP recording, the systems and methods of the present invention apply to multiphase SCS that is delivered and briefly stopped to compute a stimulation therapy dose, utilizing the same stimulation or different electrodes for recording, traditional unipolar/bipolar/multipolar SCS stimulation, or any other type of electrical stimulation of nerve fibers.

In an alternative embodiment, frequency-coded perceived stimulation strength can be measured from the variance of the ECAP signal 104. Since this signal is approximately Gaussian distributed with zero mean, the variance will change with nerve activity. Alternatively, the perceived stimulation strength can be measured from changes in the mean of the rectified and bin integrated signal (inputs to block 502).

Further, information on the active nerve fibers can be obtained from the autocorrelation function of the ECAP signal 104. From the shape of the ECAP signal 104, and using the electrodes 101 distances, nerve fiber conduction velocity can be inferred.

Using an implementation of ECAP recording, a system and process may be implemented which are able to determine an estimate of a therapeutic window on a per-subject basis. Sub-perception therapy relies on neuromodulation which does not induce a detectable ECAP in most patients, and as such the operating amplitude of this therapy is often determined as a ratio of ECAP or perception threshold, for example operating at 20% - 80% of threshold. However, the mechanism of action of sub-perception neuromodulation also relies on changes in synaptic efficacy and inhibition in the dorsal horn region of the spinal cord which may change over time during delivery of stimulation. A method of evaluating the dynamic synaptic response to stimulation is desired in order to maintain therapy amplitude within a therapeutic range, and to avoid induction of undesirable sensitization to stimulation. ECAP signals are comprised of primary Aβ dorsal column responses within the first 200 - 500 µs of stimulus, followed by later potentials which arise with recruitment of smaller diameter fibers, and evocation of post-synaptic responses. These later potentials may arise in the time frame of 400 - 2000 µs following stimuli, and with continuous stimulation, are often associated with uncomfortable levels of stimulation. Previous work has taught that these amplitudes are to be avoided, and detection of these signals in ECAP recording may be an indication of discomfort due to muscle recruitment, in particular indicating post-synaptic reflex-circuit excitation in the spinal cord.

These responses, however, may be used to advantage in a comfortable manner by the following: after establishing ECAP threshold of Aβ fibers, the threshold of Aδ and/or muscular reflex arc are determined by delivering high-amplitude single pulses which have an amplitude elevated above the standard therapeutic and ECAP determining pulse train. This amplitude is selected to evoke one or more of these later responses, and the high amplitude pulses are delivered in an isolated manner so as to not induce discomfort to the patient. This technique takes advantage of the integrative properties of the Aδ perception and reflex musculoskeletal system, which require more than a single depolarization to register discomfort. This single depolarization, however, allows measurement of the sensitivity of these fibers and of the reflex network, providing information about the plastic state of synapses within the patient's spinal cord. This, in turn, allows the sub-perception dosing to be calculated from a formula integrating not only the Aβ fiber threshold, but also Aδ dorsal column neurons as well as neuro-synaptic response sensitivity. Such information may be used to estimate and track a refined therapeutic window for a patient. For example, 80% of Aβ fiber threshold can be considered an upper bound of a sub-perception therapeutic window, while the lower bound of a sub-perception therapeutic window may be established to track a ratio, for example 10% of the late ECAP responses.

The present invention advantageously allows to provide therapeutic range estimation and neuroplastic response tracking for supra and sub-threshold neuromodulation, utilizing single isolated high amplitude impulse response measurements. Particularly, a suitable control variable is proposed to account for frequency-coded component in patient perception at high SCS frequencies. The invention further provides a signal processing based on integration that is less prone to external noise compared to pulse-by-pulse ECAP amplitude measurements, and a concurrent stimulation/recording without pausing that can measure independently the antidromic and orthodromic components of the neural response. Furthermore, a remnant SA can be dealt with based on template subtraction and adaptive filtering for clean ECAP recording. Finally, the invention provides a simple and effective closed-loop control based on dual slope modelling of a stimulation-neural dose transfer function, wherein merely simple calibration data in each patient in the most sensitive body position is needed.

Particularly, the prior art mainly focuses on closed-loop control for tonic SCS (e.g. 40 Hz) going only up to about 500 Hz as the stimulation artifact contaminates the neural response for higher frequencies. One of the main technical advantages of the invention at hand is that there is no frequency limitation given that the stimulation and recording can occur concurrently. Further, the dosage variable particularly corresponds to the actual perception sensation. Finally, the closed-loop system and method will enable dorsal root ganglion (DRG) closed-loop stimulation therapy as one does not need to record away from the stimulation site.

Further, the present invention provides a means of advanced therapeutic range and optimal amplitude closed-loop control which takes into account neurosynaptic dynamics involved in pain relief.

## Claims

1. A neurostimulation device (1), comprising a plurality of Z electrodes (200), wherein Z is an integer number and equal or larger than 3, the neurostimulation device being configured to deliver in a cycle (T) via each electrode (101.b, 101.d, 101.f) of a group of N electrodes of said plurality of Z electrodes, wherein N is an integer number being smaller or equal to Z, wherein N is equal to 3 in case Z is equal to 3, a set of electric pulses including one therapeutic electric pulse (202, 203, 204), and a number of N or N-1 charge balancing pulses (206), wherein the charge balancing pulses each have a polarity that is opposite a polarity of the therapeutic electric pulse (202, 203, 204), wherein the integrated current delivered of the therapeutic electric pulse (202, 203, 204) and charge balancing pulses is zero over time, and wherein the respective therapeutic electric pulse (202, 203, 204) comprises an amplitude (i_{TPE}),
wherein
- the neurostimulation device (1) is configured to record for the respective therapeutic electric pulse (202, 203, 204) at least one ECAP signal (104.a, 104.b, 104.c, 104.d), and wherein
- the neurostimulation device (1) comprises a closed-loop control system (800) configured to update the amplitude (i_{TPE}) of the therapeutic electric pulse (202, 203, 204) based on said ECAP signal (104.a, 104.b, 104.c, 104.d),
- wherein the closed-loop control system is configured to update said amplitude (i_{TPE}) of the therapeutic electric pulse (202, 203, 204) based on said ECAP signal (104.a, 104.b, 104.c, 104.d) such that one or more of the process variables
▪ a total therapy dose D_{Total},
▪ an antidromic therapy sensation dose D_{Anti}, or
▪ an orthodromic therapy sensation dose D_{Ortho},
- approaches a predefined set value D_{PR}, wherein the control system (800) is configured to calculate an actual value of the process variable using
▪ the antidromic ECAP signals if the process variable is D_{Anti},
▪ the orthodromic ECAP signals if the process variable is D_{Ortho}, and/or
▪ the antidromic and orthodromic ECAP signals if the process variable is D_{Total} (104.a, 104.b, 104.c, 104.d).

2. The neurostimulation device (1) according to claim 1, wherein
the ECAP signal is an antidromic ECAP signal (104.a, 104.b) and/or an orthodromic ECAP signal (104.c, 104.d), and wherein
the amplitude (i_{TPE}) of the therapeutic electric pulse (202, 203, 204) is updated based on said ECAP signal (104.a, 104.b, 104.c, 104.d) by changing an absolute amplitude value or a percentage amplitude value.

3. The neurostimulation device according to claim 1 or 2, wherein the closed-loop control system (800) is configured to subtract the actual value of the process variable from the predefined set value D_{PR} to calculate an error (e).

4. The neurostimulation device according to claim 3, wherein the control system (800) comprises a controller (C) configured to calculate the amplitude of at least one next therapeutic electric pulse (202, 203, 204), wherein the adjustment is proportional to the error multiplied with a factor (1/m).

5. The neurostimulation device according to claim 4, wherein the factor (1/m) is the inverse of a slope m of an approximation of the process variable - amplitude (i_{TPE}) transfer function.

6. The neurostimulation device according to one of the preceding claims, wherein the control system (800) is configured to approximate the process variable - amplitude (i_{TPE}) transfer function, that assigns a value of the process variable (D_{Total}; D_{Anti}; D_{Ortho}) to each value of the amplitude (i_{TPE}), by at least a first linear portion and a subsequent second linear portion, the first linear portion comprising a first slope m₁ and the second linear portion comprising a different second slope m₂, wherein the first portion includes values of the process variable smaller or equal to a threshold D_{Thr}, and the second portion includes values of the process variable above the threshold D_{Thr}.

7. The neurostimulation device according to claim 5 and according to claim 6, wherein the control system (800) comprises a processing unit (801) configured to select as said slope m the first slope m₁ in case the actual value of the process variable is below or equal to the threshold D_{Thr}, and to select as said slope m the second slope m₂ in case the actual value of the process variable is above the threshold D_{Thr}.

8. The neurostimulation device according to one of the preceding claims, wherein the neurostimulation device (1) is configured to remove a remnant stimulation artefact (SA) from the respective ECAP signal (104.a, 104.b, 104.c, 104.d) prior to calculating the actual value of the process variable.

9. The neurostimulation device according to claim 8, wherein the closed-loop control system (800) is configured to subtract a remnant stimulation artefact template from the respective ECAP signal (104.a, 104.b, 104.c, 104.d) for removing the remnant stimulation artefact (SA).

10. The neurostimulation system (1) according to one of the preceding claims, wherein the neurostimulation system (1) comprises at least two electronic circuit front-ends (105) for recording the antidromic and/or orthodromic ECAP signals (104.a, 104.b, 104.c, 104.d).

11. The neurostimulation device according to one of the claims 8 to 10, wherein in a calibration cycle the neurostimulation device (1) is configured to convert a differential output of each recording front-end (105) to a single-ended output, digitize the single-ended output, and store the digitized single-ended output, wherein the neurostimulation device (1) is configured to generate and fit a remnant stimulation artifact template (300) to the respective digitized single-ended output, and wherein during recording of the respective ECAP signal (104.a, 104.b, 104.c, 104.d), the neurostimulation device (1) is configured to convert the respective remnant stimulation artifact template (300) via a digital-to-analog converter (403) to yield an analog template and to subtract the analog template from the single-ended output containing the respective remnant stimulation artifact and ECAP signal for generating the respective ECAP signal (104.a, 104.b, 104.c, 104.d) with removed remnant stimulation artifact (SA).

12. The neurostimulation device according to one of the claims 8 to 10, wherein the neurostimulation device (1) is configured to convert a differential output of each recording front-end (105) to a single-ended output, to digitize an initial remnant stimulation artifact (SA) comprised therein by means of an analog-to-digital converter (401) and to store it as an initial template in a memory (406), wherein the neurostimulation device (1) configured to iteratively update the initial template by subtracting the single-ended output from an analog conversion of the stored template generated by an digital-to-analog converter (403) to yield a present template until an incoming remnant stimulation artifact and the present template converge within the resolution of the analog-to-digital converter (401) and digital-to-analog converter (403), wherein the present template is then subtracted from all following recorded ECAP signals (104.a, 104.b, 104.c, 104.d).

13. The neurostimulation device according to one of the claims 1 to 11, wherein the process variable corresponds to a total therapy dose D_{Total}, wherein the control system (800) is configured to calculate an actual value of process variable from the antidromic and orthodromic ECAP signals (104.a, 104.b, 104.c, 104.d) after removal of the remnant stimulation artifacts (SA) of the antidromic and orthodromic ECAP signals, by fully-wave rectifying the respective ECAP signal (104.a, 104.b, 104.c, 104.d), averaging the respective ECAP signal (104.a, 104.b, 104.c, 104.d), generating a weighted sum of the averaged antidromic ECAP signals (104.a, 104.b), wherein the weights (k_{Anti}) account for different spacings between electrodes (101.f, 101.d; 101.g, 101.f) used for recording the respective antidromic ECAP signal (104.a, 104.b), generating a weighted sum of the averaged orthodromic ECAP signals, wherein the weights (k_{Ortho}) account for different spacings between electrodes (101.a, 101.b; 101.d, 101.b) used for recording the respective orthodromic ECAP signal (104.c, 104.d), adding the two weighted sums to generate a final sum, and squaring the final sum which generates the actual value of the process variable.

14. The neurostimulation device according to one of the claims 1 to 11, wherein the process variable corresponds to an antidromic therapy sensation dose D_{Anti}, wherein the control system (800) is configured to calculate an actual value of the process variable from the antidromic ECAP signals (104.a, 104.b) after removal of the remnant stimulation artifacts (SA) from the antidromic ECAP signals, by fully-wave rectifying the respective antidromic ECAP signal (104.a, 104.b), averaging the respective antidromic ECAP signal (104.a, 104.b), generating a weighted sum of the averaged antidromic ECAP signals, wherein the weights (k_{Anti}) account for different spacings between electrodes (101.f, 101.d; 101.g, 101.f) used for recording the respective antidromic ECAP signal (104.a, 104.b), and squaring the weighted sum which generates the actual value of the process variable.

## Patentansprüche

1. Neurostimulationsvorrichtung (1), umfassend eine Mehrzahl von Z Elektroden (200), wobei Z eine ganze Zahl größer oder gleich 3 ist, wobei die Neurostimulationsvorrichtung dazu eingerichtet ist, in einem Zyklus (T) über jede Elektrode (101.b, 101.d, 101.f) einer Gruppe von N Elektroden der Mehrzahl von Z Elektroden einen Satz elektrischer Impulse abzugeben, wobei N eine ganze Zahl kleiner oder gleich Z ist, wobei N gleich 3 ist, wenn Z gleich 3 ist, wobei der Satz elektrischer Impulse einen therapeutischen elektrischen Impuls (202, 203, 204) und eine Anzahl von N oder N-1 Ladungsausgleichsimpulsen (206) umfasst, wobei die Ladungsausgleichsimpulse jeweils eine Polarität aufweisen, die einer Polarität des therapeutischen elektrischen Impulses (202, 203, 204) entgegengesetzt ist, wobei der über die Zeit integrierte abgegebene Strom des therapeutischen elektrischen Impulses (202, 203, 204) und der Ladungsausgleichsimpulse null ist und wobei der jeweilige therapeutische elektrische Impuls (202, 203, 204) eine Amplitude (i_{TPE}) aufweist, wobei
- die Neurostimulationsvorrichtung (1) dazu eingerichtet ist, für den jeweiligen therapeutischen elektrischen Impuls (202, 203, 204) mindestens ein ECAP-Signal (104.a, 104.b, 104.c, 104.d) aufzuzeichnen, und wobei
- die Neurostimulationsvorrichtung (1) ein Regelungssystem mit geschlossenem Regelkreis (800) umfasst, das dazu eingerichtet ist, die Amplitude (i_{TPE}) des therapeutischen elektrischen Impulses (202, 203, 204) auf der Grundlage des ECAP-Signals (104.a, 104.b, 104.c, 104.d) zu aktualisieren,
- wobei das Regelungssystem mit geschlossenem Regelkreis dazu eingerichtet ist, die Amplitude (i_{TPE}) des therapeutischen elektrischen Impulses (202, 203, 204) auf der Grundlage des ECAP-Signals (104.a, 104.b, 104.c, 104.d) derart zu aktualisieren, dass sich eine oder mehrere der Prozessgrößen
▪ eine Gesamttherapiedosis DTotal,
▪ eine antidrome Therapiesensationsdosis DAnti oder
▪ eine orthodrome Therapiesensationsdosis DOrtho,
- einem vordefinierten Sollwert DPR annähern, wobei das Regelungssystem (800) dazu eingerichtet ist, einen Istwert der Prozessgröße zu berechnen unter Verwendung
▪ der antidromen ECAP-Signale, wenn die Prozessgröße DAnti ist,
▪ der orthodromen ECAP-Signale, wenn die Prozessgröße DOrtho ist, und/oder
▪ der antidromen und orthodromen ECAP-Signale, wenn die Prozessgröße DTotal ist (104.a, 104.b, 104.c, 104.d).

2. Neurostimulationsvorrichtung (1) nach Anspruch 1, wobei
das ECAP-Signal ein antidromes ECAP-Signal (104.a, 104.b) und/oder ein orthodromes ECAP-Signal (104.c, 104.d) ist und wobei
die Amplitude (i_{TPE}) des therapeutischen elektrischen Impulses (202, 203, 204) auf der Grundlage des ECAP-Signals (104.a, 104.b, 104.c, 104.d) durch Ändern eines absoluten Amplitudenwerts oder eines prozentualen Amplitudenwerts aktualisiert wird.

3. Neurostimulationsvorrichtung nach Anspruch 1 oder 2, wobei das Regelungssystem mit geschlossenem Regelkreis (800) dazu eingerichtet ist, den Istwert der Prozessgröße von dem vordefinierten Sollwert DPR zu subtrahieren, um eine Regelabweichung (e) zu berechnen.

4. Neurostimulationsvorrichtung nach Anspruch 3, wobei das Regelungssystem (800) einen Regler (C) umfasst, der dazu eingerichtet ist, die Amplitude mindestens eines nächsten therapeutischen elektrischen Impulses (202, 203, 204) zu berechnen, wobei die Anpassung proportional zu der mit einem Faktor (1/m) multiplizierten Regelabweichung ist.

5. Neurostimulationsvorrichtung nach Anspruch 4, wobei der Faktor (1/m) der Kehrwert einer Steigung m einer Näherung der Übertragungsfunktion Prozessgröße-Amplitude (i_{TPE}) ist.

6. Neurostimulationsvorrichtung nach einem der vorhergehenden Ansprüche, wobei das Regelungssystem (800) dazu eingerichtet ist, die Übertragungsfunktion Prozessgröße-Amplitude (i_{TPE}), die jedem Wert der Amplitude (i_{TPE}) einen Wert der Prozessgröße (DTotal; DAnti; DOrtho) zuordnet, durch mindestens einen ersten linearen Abschnitt und einen darauf folgenden zweiten linearen Abschnitt anzunähern, wobei der erste lineare Abschnitt eine erste Steigung m1 und der zweite lineare Abschnitt eine davon verschiedene zweite Steigung m2 aufweist, wobei der erste Abschnitt Werte der Prozessgröße umfasst, die kleiner oder gleich einem Schwellenwert DThr sind, und der zweite Abschnitt Werte der Prozessgröße oberhalb des Schwellenwerts DThr umfasst.

7. Neurostimulationsvorrichtung nach Anspruch 5 und nach Anspruch 6, wobei das Regelungssystem (800) eine Verarbeitungseinheit (801) umfasst, die dazu eingerichtet ist, als die Steigung m die erste Steigung m1 auszuwählen, wenn der Istwert der Prozessgröße unterhalb oder gleich dem Schwellenwert DThr liegt, und als die Steigung m die zweite Steigung m2 auszuwählen, wenn der Istwert der Prozessgröße oberhalb des Schwellenwerts DThr liegt.

8. Neurostimulationsvorrichtung nach einem der vorhergehenden Ansprüche, wobei die Neurostimulationsvorrichtung (1) dazu eingerichtet ist, vor der Berechnung des Istwerts der Prozessgröße ein Reststimulationsartefakt (SA) aus dem jeweiligen ECAP-Signal (104.a, 104.b, 104.c, 104.d) zu entfernen.

9. Neurostimulationsvorrichtung nach Anspruch 8, wobei das Regelungssystem mit geschlossenem Regelkreis (800) dazu eingerichtet ist, zum Entfernen des Reststimulationsartefakts (SA) eine Reststimulationsartefakt-Vorlage von dem jeweiligen ECAP-Signal (104.a, 104.b, 104.c, 104.d) zu subtrahieren.

10. Neurostimulationssystem (1) nach einem der vorhergehenden Ansprüche, wobei das Neurostimulationssystem (1) mindestens zwei elektronische Schaltungs-Frontends (105) zum Aufzeichnen der antidromen und/oder orthodromen ECAP-Signale (104.a, 104.b, 104.c, 104.d) umfasst.

11. Neurostimulationsvorrichtung nach einem der Ansprüche 8 bis 10, wobei die Neurostimulationsvorrichtung (1) dazu eingerichtet ist, in einem Kalibrierungszyklus einen differentiellen Ausgang jedes Aufzeichnungs-Frontends (105) in einen massebezogenen Ausgang umzuwandeln, den massebezogenen Ausgang zu digitalisieren und den digitalisierten massebezogenen Ausgang zu speichern, wobei die Neurostimulationsvorrichtung (1) dazu eingerichtet ist, eine Reststimulationsartefakt-Vorlage (300) zu erzeugen und an den jeweiligen digitalisierten massebezogenen Ausgang anzupassen, und wobei die Neurostimulationsvorrichtung (1) während der Aufzeichnung des jeweiligen ECAP-Signals (104.a, 104.b, 104.c, 104.d) dazu eingerichtet ist, die jeweilige Reststimulationsartefakt-Vorlage (300) mittels eines Digital-Analog-Wandlers (403) umzuwandeln, um eine analoge Vorlage zu erhalten, und die analoge Vorlage von dem massebezogenen Ausgang zu subtrahieren, der das jeweilige Reststimulationsartefakt und ECAP-Signal enthält, um das jeweilige ECAP-Signal (104.a, 104.b, 104.c, 104.d) mit entferntem Reststimulationsartefakt (SA) zu erzeugen.

12. Neurostimulationsvorrichtung nach einem der Ansprüche 8 bis 10, wobei die Neurostimulationsvorrichtung (1) dazu eingerichtet ist, einen differentiellen Ausgang jedes Aufzeichnungs-Frontends (105) in einen massebezogenen Ausgang umzuwandeln, ein darin enthaltenes anfängliches Reststimulationsartefakt (SA) mittels eines Analog-Digital-Wandlers (401) zu digitalisieren und als eine anfängliche Vorlage in einem Speicher (406) zu speichern, wobei die Neurostimulationsvorrichtung (1) dazu eingerichtet ist, die anfängliche Vorlage iterativ zu aktualisieren, indem der massebezogene Ausgang von einer durch einen Digital-Analog-Wandler (403) erzeugten analogen Umsetzung der gespeicherten Vorlage subtrahiert wird, um eine aktuelle Vorlage zu erhalten, bis ein eingehendes Reststimulationsartefakt und die aktuelle Vorlage innerhalb der Auflösung des Analog-Digital-Wandlers (401) und des Digital-Analog-Wandlers (403) konvergieren, wobei die aktuelle Vorlage anschließend von allen nachfolgend aufgezeichneten ECAP-Signalen (104.a, 104.b, 104.c, 104.d) subtrahiert wird.

13. Neurostimulationsvorrichtung nach einem der Ansprüche 1 bis 11, wobei die Prozessgröße einer Gesamttherapiedosis DTotal entspricht, wobei das Regelungssystem (800) dazu eingerichtet ist, einen Istwert der Prozessgröße aus den antidromen und orthodromen ECAP-Signalen (104.a, 104.b, 104.c, 104.d) nach Entfernung der Reststimulationsartefakte (SA) der antidromen und orthodromen ECAP-Signale zu berechnen, indem das jeweilige ECAP-Signal (104.a, 104.b, 104.c, 104.d) vollweg-gleichgerichtet wird, das jeweilige ECAP-Signal (104.a, 104.b, 104.c, 104.d) gemittelt wird, eine gewichtete Summe der gemittelten antidromen ECAP-Signale (104.a, 104.b) gebildet wird, wobei die Gewichtungen (kAnti) unterschiedliche Abstände zwischen den Elektroden (101.f, 101.d; 101.g, 101.f) berücksichtigen, die zum Aufzeichnen des jeweiligen antidromen ECAP-Signals (104.a, 104.b) verwendet werden, eine gewichtete Summe der gemittelten orthodromen ECAP-Signale gebildet wird, wobei die Gewichtungen (kOrtho) unterschiedliche Abstände zwischen den Elektroden (101.a, 101.b; 101.d, 101.b) berücksichtigen, die zum Aufzeichnen des jeweiligen orthodromen ECAP-Signals (104.c, 104.d) verwendet werden, die beiden gewichteten Summen zu einer Endsumme addiert werden und die Endsumme quadriert wird, wodurch der Istwert der Prozessgröße erzeugt wird.

14. Neurostimulationsvorrichtung nach einem der Ansprüche 1 bis 11, wobei die Prozessgröße einer antidromen Therapiesensationsdosis DAnti entspricht, wobei das Regelungssystem (800) dazu eingerichtet ist, einen Istwert der Prozessgröße aus den antidromen ECAP-Signalen (104.a, 104.b) nach Entfernung der Reststimulationsartefakte (SA) aus den antidromen ECAP-Signalen zu berechnen, indem das jeweilige antidrome ECAP-Signal (104.a, 104.b) vollweg-gleichgerichtet wird, das jeweilige antidrome ECAP-Signal (104.a, 104.b) gemittelt wird, eine gewichtete Summe der gemittelten antidromen ECAP-Signale gebildet wird, wobei die Gewichtungen (kAnti) unterschiedliche Abstände zwischen den Elektroden (101.f, 101.d; 101.g, 101.f) berücksichtigen, die zum Aufzeichnen des jeweiligen antidromen ECAP-Signals (104.a, 104.b) verwendet werden, und die gewichtete Summe quadriert wird, wodurch der Istwert der Prozessgröße erzeugt wird.

## Revendications

1. Dispositif de neurostimulation (1), comprenant une pluralité de Z électrodes (200), Z étant un nombre entier supérieur ou égal à 3, le dispositif de neurostimulation étant configuré pour délivrer, au cours d'un cycle (T), par l'intermédiaire de chaque électrode (101.b, 101.d, 101.f) d'un groupe de N électrodes de ladite pluralité de Z électrodes, N étant un nombre entier inférieur ou égal à Z, N étant égal à 3 lorsque Z est égal à 3, un ensemble d'impulsions électriques comprenant une impulsion électrique thérapeutique (202, 203, 204) et un nombre de N ou N-1 impulsions d'équilibrage de charge (206), les impulsions d'équilibrage de charge ayant chacune une polarité opposée à une polarité de l'impulsion électrique thérapeutique (202, 203, 204), le courant intégré délivré de l'impulsion électrique thérapeutique (202, 203, 204) et des impulsions d'équilibrage de charge étant nul dans le temps, et l'impulsion électrique thérapeutique respective (202, 203, 204) comprenant une amplitude (i_{TPE}), dans lequel
- le dispositif de neurostimulation (1) est configuré pour enregistrer, pour l'impulsion électrique thérapeutique respective (202, 203, 204), au moins un signal ECAP (104.a, 104.b, 104.c, 104.d), et dans lequel
- le dispositif de neurostimulation (1) comprend un système de commande en boucle fermée (800) configuré pour mettre à jour l'amplitude (i_{TPE}) de l'impulsion électrique thérapeutique (202, 203, 204) sur la base dudit signal ECAP (104.a, 104.b, 104.c, 104.d),
- dans lequel le système de commande en boucle fermée est configuré pour mettre à jour ladite amplitude (i_{TPE}) de l'impulsion électrique thérapeutique (202, 203, 204) sur la base dudit signal ECAP (104.a, 104.b, 104.c, 104.d), de telle sorte qu'une ou plusieurs des variables de processus
▪ une dose thérapeutique totale DTotal,
▪ une dose de sensation thérapeutique antidromique DAnti, ou
▪ une dose de sensation thérapeutique orthodromique DOrtho,
- se rapproche d'une valeur de consigne prédéfinie DPR, le système de commande (800) étant configuré pour calculer une valeur réelle de la variable de processus à l'aide
▪ des signaux ECAP antidromiques si la variable de processus est DAnti,
▪ des signaux ECAP orthodromiques si la variable de processus est DOrtho, et/ou
▪ des signaux ECAP antidromiques et orthodromiques si la variable de processus est DTotal (104.a, 104.b, 104.c, 104.d).

2. Dispositif de neurostimulation (1) selon la revendication 1, dans lequel
le signal ECAP est un signal ECAP antidromique (104.a, 104.b) et/ou un signal ECAP orthodromique (104.c, 104.d), et dans lequel
l'amplitude (i_{TPE}) de l'impulsion électrique thérapeutique (202, 203, 204) est mise à jour sur la base dudit signal ECAP (104.a, 104.b, 104.c, 104.d) en modifiant une valeur d'amplitude absolue ou une valeur d'amplitude en pourcentage.

3. Dispositif de neurostimulation selon la revendication 1 ou 2, dans lequel le système de commande en boucle fermée (800) est configuré pour soustraire la valeur réelle de la variable de processus de la valeur de consigne prédéfinie DPR afin de calculer une erreur (e).

4. Dispositif de neurostimulation selon la revendication 3, dans lequel le système de commande (800) comprend un dispositif de régulation (C) configuré pour calculer l'amplitude d'au moins une impulsion électrique thérapeutique suivante (202, 203, 204), l'ajustement étant proportionnel à l'erreur multipliée par un facteur (1/m).

5. Dispositif de neurostimulation selon la revendication 4, dans lequel le facteur (1/m) est l'inverse d'une pente m d'une approximation de la fonction de transfert variable de processus - amplitude (i_{TPE}).

6. Dispositif de neurostimulation selon l'une des revendications précédentes, dans lequel le système de commande (800) est configuré pour approximer la fonction de transfert variable de processus - amplitude (i_{TPE}), qui attribue une valeur de la variable de processus (DTotal; DAnti; DOrtho) à chaque valeur de l'amplitude (i_{TPE}), par au moins une première portion linéaire et une seconde portion linéaire subséquente, la première portion linéaire comprenant une première pente m1 et la seconde portion linéaire comprenant une seconde pente m2 différente, la première portion comprenant des valeurs de la variable de processus inférieures ou égales à un seuil DThr, et la seconde portion comprenant des valeurs de la variable de processus supérieures au seuil DThr.

7. Dispositif de neurostimulation selon la revendication 5 et selon la revendication 6, dans lequel le système de commande (800) comprend une unité de traitement (801) configurée pour sélectionner, en tant que ladite pente m, la première pente m1 lorsque la valeur réelle de la variable de processus est inférieure ou égale au seuil DThr, et pour sélectionner, en tant que ladite pente m, la seconde pente m2 lorsque la valeur réelle de la variable de processus est supérieure au seuil DThr.

8. Dispositif de neurostimulation selon l'une des revendications précédentes, dans lequel le dispositif de neurostimulation (1) est configuré pour supprimer un artefact de stimulation résiduel (SA) du signal ECAP respectif (104.a, 104.b, 104.c, 104.d) avant le calcul de la valeur réelle de la variable de processus.

9. Dispositif de neurostimulation selon la revendication 8, dans lequel le système de commande en boucle fermée (800) est configuré pour soustraire un gabarit d'artefact de stimulation résiduel du signal ECAP respectif (104.a, 104.b, 104.c, 104.d) afin de supprimer l'artefact de stimulation résiduel (SA).

10. Système de neurostimulation (1) selon l'une des revendications précédentes, dans lequel le système de neurostimulation (1) comprend au moins deux étages d'entrée de circuit électronique (105) pour enregistrer les signaux ECAP antidromiques et/ou orthodromiques (104.a, 104.b, 104.c, 104.d).

11. Dispositif de neurostimulation selon l'une des revendications 8 à 10, dans lequel, au cours d'un cycle d'étalonnage, le dispositif de neurostimulation (1) est configuré pour convertir une sortie différentielle de chaque étage d'entrée d'enregistrement (105) en une sortie asymétrique, numériser la sortie asymétrique et stocker la sortie asymétrique numérisée, le dispositif de neurostimulation (1) étant configuré pour générer et ajuster un gabarit d'artefact de stimulation résiduel (300) à la sortie asymétrique numérisée respective, et dans lequel, pendant l'enregistrement du signal ECAP respectif (104.a, 104.b, 104.c, 104.d), le dispositif de neurostimulation (1) est configuré pour convertir le gabarit d'artefact de stimulation résiduel respectif (300), au moyen d'un convertisseur numérique-analogique (403), afin d'obtenir un gabarit analogique et pour soustraire le gabarit analogique de la sortie asymétrique contenant l'artefact de stimulation résiduel respectif et le signal ECAP afin de générer le signal ECAP respectif (104.a, 104.b, 104.c, 104.d) dont l'artefact de stimulation résiduel (SA) a été supprimé.

12. Dispositif de neurostimulation selon l'une des revendications 8 à 10, dans lequel le dispositif de neurostimulation (1) est configuré pour convertir une sortie différentielle de chaque étage d'entrée d'enregistrement (105) en une sortie asymétrique, numériser un artefact de stimulation résiduel initial (SA) qu'elle comprend au moyen d'un convertisseur analogique-numérique (401) et le stocker en tant que gabarit initial dans une mémoire (406), le dispositif de neurostimulation (1) étant configuré pour mettre à jour de manière itérative le gabarit initial en soustrayant la sortie asymétrique d'une conversion analogique du gabarit stocké générée par un convertisseur numérique-analogique (403), afin d'obtenir un gabarit courant, jusqu'à ce qu'un artefact de stimulation résiduel entrant et le gabarit courant convergent dans les limites de la résolution du convertisseur analogique-numérique (401) et du convertisseur numérique-analogique (403), le gabarit courant étant ensuite soustrait de tous les signaux ECAP enregistrés ultérieurement (104.a, 104.b, 104.c, 104.d).

13. Dispositif de neurostimulation selon l'une des revendications 1 à 11, dans lequel la variable de processus correspond à une dose thérapeutique totale DTotal, le système de commande (800) étant configuré pour calculer une valeur réelle de la variable de processus à partir des signaux ECAP antidromiques et orthodromiques (104.a, 104.b, 104.c, 104.d), après suppression des artefacts de stimulation résiduels (SA) des signaux ECAP antidromiques et orthodromiques, en effectuant un redressement double alternance du signal ECAP respectif (104.a, 104.b, 104.c, 104.d), en moyennant le signal ECAP respectif (104.a, 104.b, 104.c, 104.d), en générant une somme pondérée des signaux ECAP antidromiques moyennés (104.a, 104.b), les poids (kAnti) tenant compte de différents espacements entre les électrodes (101.f, 101.d; 101.g, 101.f) utilisées pour enregistrer le signal ECAP antidromique respectif (104.a, 104.b), en générant une somme pondérée des signaux ECAP orthodromiques moyennés, les poids (kOrtho) tenant compte de différents espacements entre les électrodes (101.a, 101.b; 101.d, 101.b) utilisées pour enregistrer le signal ECAP orthodromique respectif (104.c, 104.d), en additionnant les deux sommes pondérées pour générer une somme finale, et en élevant la somme finale au carré, ce qui génère la valeur réelle de la variable de processus.

14. Dispositif de neurostimulation selon l'une des revendications 1 à 11, dans lequel la variable de processus correspond à une dose de sensation thérapeutique antidromique DAnti, le système de commande (800) étant configuré pour calculer une valeur réelle de la variable de processus à partir des signaux ECAP antidromiques (104.a, 104.b), après suppression des artefacts de stimulation résiduels (SA) des signaux ECAP antidromiques, en effectuant un redressement double alternance du signal ECAP antidromique respectif (104.a, 104.b), en moyennant le signal ECAP antidromique respectif (104.a, 104.b), en générant une somme pondérée des signaux ECAP antidromiques moyennés, les poids (kAnti) tenant compte de différents espacements entre les électrodes (101.f, 101.d; 101.g, 101.f) utilisées pour enregistrer le signal ECAP antidromique respectif (104.a, 104.b), et en élevant la somme pondérée au carré, ce qui génère la valeur réelle de la variable de processus.
